# EUROPEAN PATENT APPLICATION

(11) **EP 4 623 960 A1**
(43) Date of publication of application: **01.10.2025**
(21) Application number: 24166504.1
(22) Date of filing: 26.03.2024
(51) Int. Cl.: A61M 5/31, A61M 5/315, A61M 5/32, A61M 5/20

(54) **AUTOINJECTOR FOR DISPENSING A LIQUID PRODUCT**

(71) Applicant: Ypsomed AG, 3401 Burgdorf (CH)
(72) Inventor: Maechler, Silas, 3270 Aarberg (CH); Labudde, Marc, 3012 Bern (CH); Schrul, Christian, 3414 Oberburg (CH)
(74) Representative: Meier Obertüfer, Jürg

(57) **Abstract**

The present application relates to an autoinjector for dispensing a liquid product, particularly a drug. The autoinjector comprises a housing and a product container arranged in the housing. The product container preferably is a syringe The product container comprises a displaceable piston, which is displaceable in a dispensing direction for dispensing a liquid product contained in the product container. The autoinjector further includes a propulsion means which acts on the piston during liquid product discharge. An injection needle is in fluid communication with the product container and at least partially protrudes from the housing through an opening provided at a distal end thereof. The autoinjector comprises at least one detection element (20.1, 20.2) for detecting the presence of the liquid product on an outside of the injection needle or on the housing after liquid product discharge.

## Description

### Technical Field

The invention relates to an autoinjector, often also referred to as an auto-injection device, with which a liquid product contained in a product container can be automatically dispensed after triggering, especially through an injection needle. The liquid product is particularly a drug. More particularly, the invention relates to an autoinjector which allows for a better recognition of failures during the administration of an injection, especially the incurrence of so-called wet injections or air-shots.

### Background Art

Auto-injectors are known from the prior art, such as from EP2742962 A2, to deliver an injection easily and safely with a preloaded spring.

When administering a drug by means of an autoinjector, it is important to determine if the entire dose of the drug was correctly administered to a patient, as the outcome of the treatment by the drug highly depends on the adherence to a dosage regimen. Hence, autoinjectors usually include some sort of feedback to let a patient know when the dose of the drug has been fully administered, such as for example a visual, audible, or tactical feedback. However, such feedback only indicate that the entire dose of the drug has been dispensed by the autoinjector but provide no indication on whether the full dose has indeed been injected. In practice, it often occurs that the autoinjector is removed prematurely from an injection site, i.e. before the full dose has been administered, resulting in a so-called air-shot. In other instances, a part of the dose may flow or leak (also referred to as back-flow or reflux) onto the skin of a patient due to backflow generated by tissue back pressure, an incomplete insertion of a tip of an injection needle into skin of a patient or wrong manipulation of the autoinjector by a patient, resulting in a so-called wet injection.

Hence, there is the need to provide an autoinjector which can reliably detect the correct administration, i.e. injection of a drug to a patient. Several solutions trying to solve this problem are known. However, these solutions rely on indirect methods to detect wet injections or air-shots, such as pressure sensors, contact sensors, timers, or the like.

For example, WO 2019/079474 A1 discloses an injection training device which can detect the correct placement of the injection training device on a contact site of a user during an injection training event. The injection training device is configured to detect an error condition if an actuation member is actuated while the sensor does not detect contact of the injection training device with a contact site.

WO 2020/154170 A1 describes a drug delivery system comprising a pressure sensor configured to detect at least tissue back pressure during use of the drug delivery system.

Hence, none of the solutions known in the art can directly detect the presence of a liquid product, particularly a drug, which was not correctly injected to a patient.

The article Poulin, A., Aeby, X. & Nyström, G. Water activated disposable paper battery. Sci Rep 12, 11919 (2022). https://doi.org/10.1038/s41598-022-15900-5 describes a water-activated paper battery comprising a zinc anode and a graphite cathode which are printed on a paper substrate. The battery is activated by water, which acts as electrolyte and allows a current to flow. Such batteries are commercially available e.g. from fuelium (https://fuelium.tech/).

### Summary of the invention

It is the object of the invention to create an autoinjector which allows a reliable and efficient identification of wet injections and air-shots thus ensuring the correct adherence to a dosage regimen during a treatment.

The solution of the invention is specified by the features of claim 1. According to the invention, an autoinjector for dispensing a liquid product, particularly a drug, comprises a housing adapted to receive a product container. The product container preferably is a syringe. The product container comprises a displaceable piston, which is displaceable in a dispensing direction for dispensing a liquid product contained in the product container. The autoinjector further includes a propulsion means which acts on the piston during liquid product discharge. An injection needle is in fluid communication with the product container and at least partially protrudes from the housing through an opening provided at a distal end thereof. The autoinjector comprises at least one detection element for detecting the presence of the liquid product on an outside of the injection needle or on the housing after liquid product discharge.

As a wet injection or air-shot results in a part of the liquid product to moisten an outside of the injection needle or a part of the housing, the at least one detection element allows to reliably detect if a wet injection or air-shot has occurred. Contrary to the solutions known in the art, the inventive configuration of the autoinjector allows for a direct detection of the liquid product which failed to be correctly injected. This greatly enhances the reliability in detecting wet injections and air-shots.

The term "drug" as used herein comprises any flowable medical formulation suitable for controlled administration through a means such as a cannula or injection needle, for example comprising a liquid, a solution, a gel, or a fine suspension containing one or more active medical ingredients. A medical formulation may comprise a single active ingredient or be a mixed or co-formulated composition comprising a plurality of active ingredients. A medical formulation may comprise drugs such as peptides (e.g., insulins, insulin-containing preparations, GLP-1-containing, GLP-1-derived or GLP-1-analogous preparations), proteins, hormones, biologically obtained or artificial active ingredients, active ingredients based on hormones or genes, nutrient formulations, enzymes and further substances both in solid (suspended) or liquid form but also polysaccharides, vaccines, DNA, RNA, or other oligonucleotides, antibodies or parts of antibodies, and suitable bases, auxiliary and excipient substances.

The housing preferably has an elongated shape. Preferably, the housing is sleeve-shaped and/or cylindrical, in particular circular cylindrical. The housing thereby includes a longitudinal axis. Alternatively, the housing may be of any other suitable shape, such as cuboid or any other polyhedron shape.

The housing includes a distal end and a proximal end. As used herein, the distal end is the end of the housing which faces skin of a patient during liquid product discharge and the proximal end is the end of the housing opposite of the skin of a patient during liquid product discharge. Preferably, the distal and proximal ends of the housing are located along the longitudinal axis of the housing, i.e. in the case of a cylindrical housing, the proximal and distal ends correspond to the two base areas of the cylinder.

The housing encloses an inner space. At least the distal end of the housing is preferably open, such as to allow the injection needle to protrude out of the housing at least during liquid product discharge.

The housing is preferably made of a polymer material. Use of a polymer material simplifies the production of the housing, as the housing may be injection molded.

The product container is arranged in the inner space of the housing, preferably in such a manner that it cannot be displaced along the longitudinal axis of the housing, i.e. in an axially fixed manner. Preferably, this is achieved by means of a product container holder, which mechanically holds the product container in the axially fixed manner relative to the housing. The product container holder is preferably fixedly attached to an inner wall of the housing or is a protrusion formed on the inner wall of the housing. In this case, a tip of the injection needle preferably protrudes out of the distal end of the housing. This allows the needle to be inserted into a puncture site of a patient by means of a movement of the housing towards the patient.

Preferably, when the product container is arranged within the housing in the axially fixed manner, a needle protection sleeve is provided, which forms the distal end of the autoinjector and has an opening for the injection needle. The needle protection sleeve is movable relative to the housing in the direction of the longitudinal axis thereof. The needle protection sleeve preferably is arranged in an initial position such that the protection sleeve entirely covers the injection needle. The needle protection sleeve is displaceable relative to the housing from its initial position in the proximal direction by an actuating stroke into an actuated position. In the actuated position the tip of the injection needle protrudes from a distal end or through the opening of the needle protection sleeve. Preferably, the needle protection sleeve may subsequently be displaced, e.g. by a needle protection stroke, distally relative to the housing from the actuated position into a needle protection position. In the needle protection position, the distal end of the needle protection sleeve is located distally relative to the tip of the injection needle, i.e. the needle protection sleeve completely covers the injection needle, in order to prevent a risk of injury from an exposed tip of the injection needle after the autoinjector has been used or after the product discharge. The needle protection sleeve is preferably moved against the force of a at least one second spring, which can be referred to as needle shielding spring, in the proximal direction. The at least one second spring preferably moves the needle protection sleeve from the actuated position into the needle protection position.

Preferably, the autoinjector comprises a locking member, which, for example, is resiliently arranged at the distal end of the housing and locks the needle protection sleeve in the needle protection position. This means that the locking member prevents the needle protection sleeve to be again moved in the proximal direction or to be moved from the needle protection position only to such an extent that the tip of the injection needle does not protrude from the distal end of the needle protection sleeve.

Alternatively, the product container may be displaceable arranged within the housing, i.e. such that it can be linearly displaced along the longitudinal axis of the housing and relative thereto, preferably at least in the direction of the distal end of the housing. In this case, the injection needle is preferably directly coupled to a distal end of the product container, such that displacement of the product container in the distal direction relative to the housing allows a tip of the injection needle to emerge from an opening at the distal end of the housing, thus enabling for an automatic insertion of the needle tip into skin of a patient. In this embodiment, the product container preferably is movable relative to the housing in the proximal direction, such as to move the tip of the injection needle back into the housing.

The product container preferably is filled with the liquid product. Alternatively, the product container comprises means to allow the filling of the liquid product into the product container, such as e.g. a port or membrane.

The product container preferably has a volume of less than 5 ml, more preferably of less than 2.5 ml, most preferably of less than 1 ml. Most preferably, the product container has a volume of between 0.1 ml and 3 ml, more preferably of 0.5ml to 2.5ml. The product container preferably is made of glass or a polymer material.

The product container comprises at least one outlet through which the liquid product may flow during product discharge. The injection needle is preferably in fluid communication with the at least one outlet. Most preferably, the injection needle is directly arranged on the at least one outlet of the product container. Alternatively, the injection needle may be in fluid communication with the at least one outlet by means of a duct or the like. Preferably, the injection needle is arranged to be parallel to the longitudinal axis of the housing. Most preferably, the injection needle is arranged on the longitudinal axis of the housing.

The product container comprises a piston which is displaceable arranged therein, such that the liquid product is pushed out of the product container when the piston is displaced in the dispensing direction. Preferably, the dispensing direction is parallel to the longitudinal axis of the housing and oriented towards the distal end of the housing.

Preferably, the product container is configured as a syringe, which has a syringe body at the distal end of which the injection needle is firmly arranged. Hence, when the product container is configured as a syringe, the product container and the injection needle form a single syringe module. The syringe body is preferably cylindrical and surrounds a volume, within which the piston is arranged. In the context of syringes, the piston is also usually referred to as plunger. The piston seals against the inside diameter of the syringe body and is displaceable with respect to the syringe body in the dispensing direction, i.e. towards a distal end of the syringe body for product discharge. The liquid product is arranged between the piston and the injection needle prior to product discharge and preferably in the delivery state of the autoinjector. By the displacement of the piston relative to the syringe body in the dispensing direction, the liquid product is pushed out of the product container and into the injection needle. The syringe body may have a flange, which may also be referred to as a finger flange, at its proximal end, i.e. the rear end or the end opposite the injection needle. A syringe designed in this way is available as a standard syringe, so there is no need to use a specially adapted syringe for the autoinjector.

The propulsion means preferably is in active connection with the piston of the product container. Preferably, the propulsion means comprises at least one actor which exerts a force in the dispensing direction on the piston of the product container. Preferably, the at least one actor includes a pre-tensioned first spring, such as for example a helical spring or a torsion spring. Alternatively, the at least one actor may be a pre-tensioned elastic element, an electromechanical actor, or any other suitable type. The propulsion means preferably comprises a drive element onto which the at least one actor is able to exert a force in the dispensing direction. The drive element preferably is sleeve shaped and arranged within the inner space of the housing in a linearly displaceable manner, preferably in the direction of the longitudinal axis of the housing. The drive element preferably rests on the piston of the product container with a distal end thereof.

Hence, the drive element may be used to deliver a discharge stroke on the piston in order to push out the liquid product from the product container and through the injection needle. The drive element is preferably coupled to a trigger element, such as for example a button, located on an outside of the housing, which is actionable by a patient in order to trigger the discharge stroke. Alternatively, the trigger element may be actionable by a movement of a needle protection sleeve, as described above.

The at least one detection element is preferably arranged on or in the vicinity of the distal end of the housing or on the needle protection sleeve in the case that the autoinjector comprises a needle protection sleeve. Alternatively or additionally thereto, the at least one detection element may be in contact with an outside of the injection needle after product discharge, preferably moved into contact with the outside of the injection needle after product discharge.

The at least one detection element may comprise at least one chemical compound which undergoes a chemical reaction in the presence of the liquid product, preferably a chemical reaction that yields a visible effect, such as e.g. a change of color. Hence, a patient may visibly detect the presence of the liquid product on the housing or needle protection sleeve by visually checking the at least one detection element.

Preferably, the at least one detection element is a liquid sensitive element which is in communication with an electronic module in or on the housing. In this embodiment, the at least one detection element is configured to detect the presence of the liquid product, preferably of the water comprised in the liquid product. Hence, the at least one detection element may for example be a water or moisture sensor element. In the case that the liquid product comprises at least one salt or other ionic component, the at least one detection element might be a conductivity sensor which is able to detect an increase of conductivity due to the presence of the liquid product on the at least one detection element.

The electronic module preferably comprises at least one integrated circuit or microcontroller which is able to process a signal received from the at least one detection element as well as a printed circuit board. Preferably, the electronic module comprises or is connected to an output means to signal a patient the detection of the liquid product on the outside of the injection needle or on the housing after liquid product discharge, such as e.g. an indicator light, preferably in the form of a light emitting diode, a sound generator, a small liquid crystal display or the like. The electronic module may further comprise an electric power source, such as a battery, in order to power the electronic module and - if required - to provide electric power to the at least one detection element. If the autoinjector itself comprises an electric power source, the electronic module may be alternatively connected to the electric power source of the autoinjector. The at least one detection element is preferably connected to the electronic module by means of wires. If the autoinjector itself comprises electronics, the electronic module may also be integrated in the electronics of the autoinjector.

More preferably, the at least one detection element is a paper-based battery. Paper based batteries are activated by water and usually use Zinc as a biodegradable metal anode, graphite as a nontoxic cathode material and paper as a biodegradable substrate. Paper based batteries remain inactive until water is provided and absorbed by the paper substrate, taking advantage of its natural wicking behaviour.

Use of a paper-based battery as the at least one detection element has the advantage that the signal generated by the at least one detection element simply is electric current, i.e. a voltage. Hence, the electronic module may be construed in a simple manner, e.g. by a single component which is provided with an electric current only when the at least one detection unit configured as paper-based battery has been moistened with the liquid product. The electronic module may thus for example be a single light emitting diode, a buzzer or any other component which emits a signal when provided with an electric current.

Preferably, the at least one detection element is arranged on a movable arm element, said movable arm element being movable relative to the housing to bring the at least one detection element in proximity or in physical contact with an outer surface of the injection needle after liquid product discharge. In this context, proximity to the injection needle implies that a distance between the detection element and the injection needle is smaller than a typical dimension or size of a droplet of the liquid product.

With this embodiment, the at least one detection element may detect any of the liquid product flowing along the injection needle or adhering thereon after product discharge. Any such liquid product will inherently be the result of a wet injection or of an air-shot, as normally the entire liquid product present in the liquid container is injected into a patient under normal circumstances, i.e. when an injection with the autoinjector has been carried out without error.

Preferably, the at least one arm element is moved from a retracted position into a contact position after product discharge. In the retracted position, the at least one arm element preferably is located inside the housing or inside a needle protection sleeve, and laterally spaced apart from the injection needle. In the contact position, the at least one arm element is at least partially in physical contact with the injection needle, especially with a distal end. The at least one detection element is located such on the at least one arm element, that it is in physical contact with the injection needle in the contact position. Preferably, the at least one detection element is located on a distal end of the at least one arm element. In the contact position, the at least one arm element preferably is still located inside the housing or the needle protection sleeve but may also protrude distally out of the housing or needle protection sleeve. The at least one arm element is displaced at least in a lateral direction, i.e. a direction which is perpendicular to the extension of the injection needle, during a movement from the retracted position into the contact position in order to be brought in contact with an outside of the injection needle in the contact position. Preferably, when moving from the retracted position to the contact position, the at least one arm element is also moved in a distal direction relative to the housing. This means that the displacement direction of the at least one arm element has a component in the distal direction relative to the housing as well as in the lateral direction relative to the injection needle when moving from the retracted position into the contact position.

In a preferred embodiment, the at least one arm element is made of a flexible material, preferably of a flexible polymer material, and is arranged to be parallel to the longitudinal axis of the housing. As in most cases the injection needle is parallel to the longitudinal axis of the housing or arranged thereon, the at least one arm element is thus also parallel to the injection needle. In this embodiment, the at least one arm element is mounted on an inside wall of the housing or of a needle protection sleeve in a linearly movable manner, i.e. the at least one arm element is displaceable relative to the housing or the needle protection sleeve in a direction parallel to the longitudinal axis of the housing. The housing or the needle protection sleeve comprises at least one guide element arranged on an inside wall thereof, the at least one guide element including a ramp portion which deflects a distal end of the at least one arm element towards the injection needle when the at least one arm element is moved from the retracted position into the contact position.

The movement of the at least one arm element from the retracted position into the contact position may be coupled to the discharge stroke, i.e. to the distal movement of the drive element such that the at least one arm element is moved from the retracted position into the contact position during liquid product dispense. The at least one arm element may be moved from the retracted position into the contact position at the same time the piston is moved in the discharge direction, e.g. by mechanically coupling the at least one arm element with the drive element or the piston.

In a further alternative, in the case that the autoinjector comprises a needle protection sleeve, the movement of the at least one arm element from the retracted position into the contact position may be coupled to the movement of the needle protection sleeve from the needle protection position to the actuated position, or from the actuated position into the needle protection position. This may e.g. be achieved by mechanically coupling the at least one arm element with the at least one second spring.

Preferably, the autoinjector comprises more than one arm element, wherein at least one detection element is arranged on each of the more than one arm elements. Preferably, the autoinjector comprises two, three, four or more arm elements. In the case that the autoinjector comprises more than one arm element, the arm elements are preferably distributed evenly around the injection needle. For example, if the autoinjector comprises n arm elements, each arm element is arranged at an angle of 360°/n from its neighboring arm elements.

In the case that the autoinjector comprises more than one arm element, all arm elements are preferably moved synchronously from the retracted position into the contact position.

Preferably, the housing comprises a needle protection sleeve arranged at the distal end of the housing and surrounding the injection needle. The needle protection sleeve is linearly movable along a longitudinal axis of the housing and relative to the housing. The at least one detection element is arranged on the needle protection sleeve.

The needle protection sleeve is preferably configured as described above. Provision of the at least one detection element on the needle protection sleeve ensures that the at least one detection element is placed a at distal most position of the autoinjector.

Preferably, the at least one detection element is arranged on an inside wall of the needle protection sleeve, preferably at a distal end thereof. Provision of the at least one detection element on an inside wall of the needle protection sleeve allows to keep the at least one detection element safe from any damage, contamination, or liquid contact prior to product discharge.

Preferably, the at least one detection element is arranged on at least one movable support element, the at least one movable support element being arranged within the housing and being movable from a first position, in which at least a part of the at least one movable support element is positioned distally in front of the injection needle, to a second position, in which the at least one movable support element is positioned laterally relative to the injection needle, and back to the first position.

The part of the at least one movable support element is spaced apart from the injection needle along the longitudinal axis of the housing, especially spaced apart from the tip of the injection needle, in the first position. This means that the at least one moveable support element does not contact the injection needle in the first position. The part of the at least one moveable support element is preferably positioned directly distally of the tip of the injection needle, i.e. the part of the at least one moveable support element intersects an imaginary axis including a longitudinal axis of the injection needle. Preferably, the part of the at least one moveable support element which lies distally in front of the injection needle is a distal end of the at least one moveable support element.

In the second position, the at least one moveable support element is positioned laterally relative to the injection needle, i.e. the at least one moveable support element, preferably a distal end thereof, is positioned at a location which is spaced apart from an imaginary axis including a longitudinal axis of the injection needle in a direction perpendicular thereto. Hence, in the second position, the at least one moveable support element does not obstruct the injection needle in the distal direction, so that the injection needle may be inserted into skin of a patient.

Preferably, the autoinjector comprises more than one moveable support element, such as e.g. two, three, four or more moveable support elements. In this case, the movable support element are preferably configured to close the opening of the housing, preferably to completely close the opening of the housing. As such, the moveable support elements form a needle protection cover preventing an accidental pricking of a patient and ensuring the protection of the injection needle from any contamination. In this case, the moveable support elements are preferably distributed evenly around the injection needle. For example, if the autoinjector comprises m moveable support elements, each support element is arranged at an angle of 360°/m from its neighboring moveable support elements. This ensures a complete closing of the opening.

The at least one detection element is preferably arranged on the part of the at least one movable support element which lies distally in front of the injection needle in the first position. Preferably, the at least one detection element is arranged at a distal end of the at least one moveable support element.

Preferably, the at least one moveable support element is configured to be displaced laterally relative to the injection needle and proximally relative to the housing while moving from the first position into the second position. This allows to move the at least one moveable support element away of a tip of the injection needle in the proximal direction, such that the tip of the injection needle may be inserted into skin of a patient without the need to move the injection needle into the distal direction relative to the housing.

The at least one moveable support element is preferably configured to move from the first position to the second position prior to product discharge, i.e. prior to the discharge stroke. Further preferably, the at least one moveable support element is configured to move from the second position back to the second position after product discharge.

The movement of the at least one moveable support element from the first position into second position is preferably coupled to the discharge stroke, i.e. to the distal movement of the drive element such that the at least one moveable support element is moved from the first position into the second position prior to the discharge of the liquid product. In order to achieve this sequential movement of the at least one moveable support element and the piston, the drive element is preferably spaced apart from the piston in a delivery state of the autoinjector, while the at least one moveable support element is mechanically coupled to the drive element such that a movement of the drive element in the distal direction results in the lateral displacement of the at least one support element relative to the injection needle and preferably also a displacement thereof in the proximal direction relative to the housing.

Preferably, the autoinjector comprises a mechanism which is configured to move the at least one movable support element from the first position to the second position when the needle protection sleeve is moved in a proximal direction relative to the housing and to move the at least one movable support element from the second position back to the first position when the needle protection sleeve is moved in a distal direction relative to the housing.

This coupling of the movements of the needle protection sleeve with the movement of the at least one moveable support element may for example be achieved by means of a carrier element located on the inside of the needle protection sleeve and which is in engagement with a groove or rib located on the at least one moveable support element, such that the carrier element entrains the at least one moveable support element when moving from the initial position to the actuated position and subsequently into the needle protection position.

Preferably, the autoinjector comprises a wireless communication element which is configured to transmit a signal when the at least one detection element detects the presence of a liquid.

Hence, detection of a wet injection or air-shot by means of the at least one detection device may be registered and/or displayed on a remote device, e.g. a mobile device such as a tablet computer, mobile phone or the like.

Preferably, the at least one wireless communication element is a Bluetooth^{®} module or a Bluetooth^{®} low energy module. Alternatively, the at least one wireless communication element may be of any other suitable type, such as a radiofrequency communication module, an infrared communication module or the like.

If the at least one detection module is configured as a paper-based battery, the voltage provided by the paper-based battery upon contact with the liquid product powers the at least one wireless communication device on, such that the at least one wireless communication device is able to emit a wireless signal.

Other advantageous embodiments and combinations of features come out from the detailed description below and the entirety of the claims.

### Brief description of the drawings

The drawings used to explain the embodiments show:
- Figs. 1a -c: schematic longitudinal sectional views of a first embodiment of an autoinjector according to the present invention;
- Figs. 2a - c: schematic longitudinal sectional views of a second embodiment of an autoinjector according to the present invention;
- Figs. 3a -c: schematic longitudinal sectional views of a third embodiment of an autoinjector according to the present invention;

In the figures, the same components are given the same reference symbols.

### Preferred embodiments

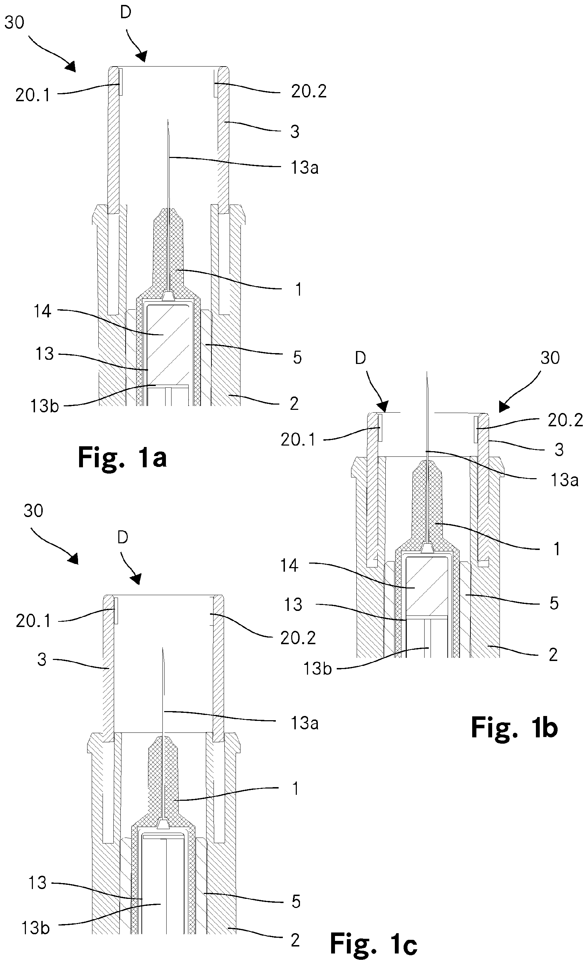

Figs. 1a, 1b and 1c schematically show longitudinal sectional views of a first embodiment of an autoinjector. In the figures, only the distal end D of the autoinjector 30 is shown in a simplified manner.

Fig. 1a shows the autoinjector 30 prior to product discharge. The needle protection sleeve 3 is in its initial distal position. As may be seen in this figure, the needle protection sleeve is slidable in the proximal direction relative to the housing 2 to allow a tip of the injection needle 13a to protrude therefrom (as shown in Fig. 1b). Further, the product container holder 5 and the product container 13 are all arranged within the housing 2. The liquid product 14, for example a liquid drug, is filled within the container 13 between the injection needle 13a and the piston 13b. In the embodiment shown, the injection needle 13a is fixedly attached to a distal end of the product container 13 on an opening thereof.

Fig. 1b shows the autoinjector during product discharge. The needle protection sleeve has been moved in proximal direction from the initial position into an actuated position by pushing the autoinjector towards the skin of a patient (not shown) at an injection site. Thereby, the tip of the injection needle protrudes distally therefrom. The tip of the injection needle 13a may hence prick the skin of the patient in order to inject the liquid product subcutaneously into the patient. The movement of the needle protection sleeve 3 into the actuated position triggers product discharge, i.e. the piston 13b is being moved in the distal, i.e. dispensing direction by means of a first spring.

Fig. 1c shows the autoinjector 30 after product discharge. Once the liquid product 14 has been fully dispensed, i.e. discharged, the autoinjector is removed from the injection site and the needle protection sleeve 3 is moved distally by means of a second spring into a needle protection position, as shown. The needle protection sleeve 3 protects the patient and any other person from accidental pricking when in the needle protection position and is preferably locked into this position to avoid any unwanted movement into the proximal direction, which may lead to the tip of the injection needle 13a to protrude again distally over the needle protection sleeve 3.

The autoinjector 30 comprises two detection elements 20.1, 20.2 which are arranged on an inside of the needle protection sleeve 3 in an area of the distal end thereof. At least part of the liquid product not correctly injected into a patient, e.g. as a result of a wet injection or air-shot, will contact and wet at least one of the two detection elements 20.1, 20.2. The at least one wetted detection element 20.1, 20.2 will generate a signal for an electronic module in order to inform the patient about the occurred error. Alternatively, the two detection elements 20.1, 20.2 may also be inspected visually by the patient in case that the detection elements 20.1, 20.2 undergo a colour change upon contact with the liquid product or when wetted. Instead of two oppositely arranged detection elements, a single detection element may be present, spanning or covering the inner circumference of the needle protection sleeve in an area of the distal end thereof.

In the case that the detection element 20 is a paper-based battery, this wetting will activate the paper-based battery which hence induces a current which may be detected by the electronic module. Likewise, if the detection element 20 is a moisture sensor or other liquid detection sensor, this sensor will generate an appropriate signal when wetted, said signal being transmitted to the electronic element. The electronic element may in both cases emit a signal or transmit a signal to a remote device to inform the patient about the injection error.

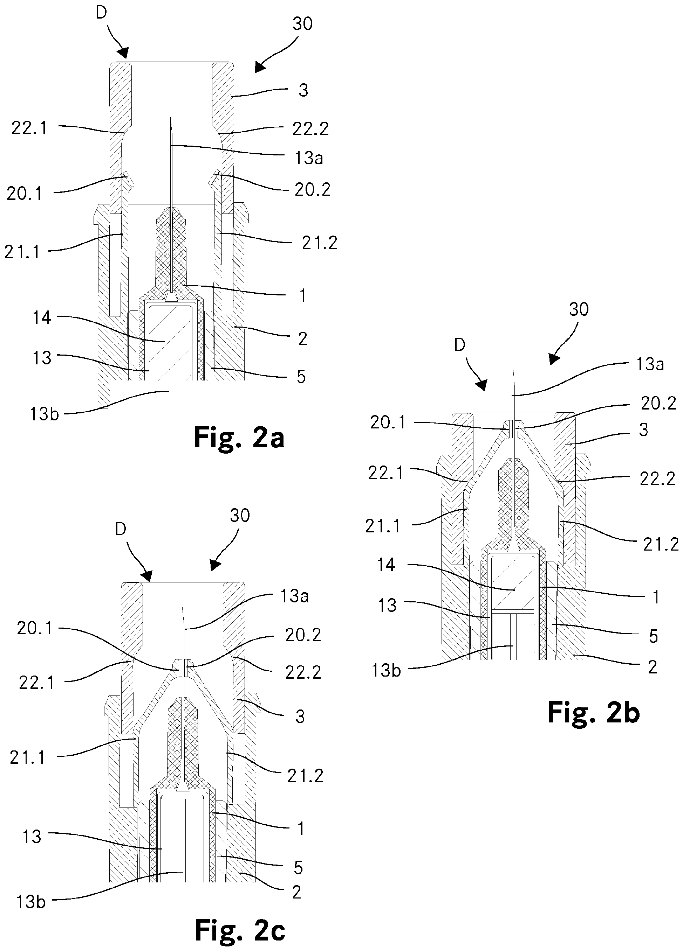

Figs. 2a, 2b and 2c schematically show longitudinal sectional views of a second embodiment of a distal end D of an autoinjector 30 in a simplified manner. The autoinjector 30 comprises two detection elements 20.1, 20.2 each located at a distal end of a flexible or deformable arm element 21.1, 21.2. The arm elements 21.1, 21.2 are arranged on an inside of the housing and at least partially protrude into an inside of the needle protection sleeve 3 when the latter is in the initial position. Again, Fig. 2a shows the autoinjector 30 prior to product discharge.

Fig. 2b shows the second embodiment of the autoinjector 30 during product discharge. Similar to the first embodiment described above, the needle protection sleeve 3 is moved in the proximal direction by pushing the autoinjector 30 onto skin of a patient. In this embodiment, the needle protection sleeve 3 comprises two ramps 22.1, 22.2 located on an inside wall of the needle protection sleeve 3, the two ramps 22.1, 22.2 are located directly in the distal direction of the two arm elements 21.1, 21.2. Hence, when the needle protection sleeve 3 is moved in the proximal direction, the distal ends of the two arm elements 21.1, 21.2 will contact the ramps 22.1, 22.2 and be deflected towards the injection needle 13a. The length of the two arm elements 21.1, 21.2 are chosen such that the distal end of the two arm elements 21.1, 21.2 with the detection elements 20.1, 20.2 contact an outside surface of the injection needle 13a when the needle protection sleeve 3 is in the actuated position. Further, the length of the arm elements 21.1, 21.2 is chosen such that the distal ends thereof contact the outside of the injection needle 13a in an area which is close to the distal end D of the needle protection sleeve 3 when the latter is in the actuated position. Thereby, any of the liquid product 14 which is incorrectly discharged, i.e. which results from a wet injection or an air-shot, will contact and subsequently wet the detection elements 20.1, 20.2.

Fig. 2c shows the autoinjector 30 after product discharge. In the embodiment shown, the two arm elements 21.1, 21.2 are deformable and stay in contact with the outside of the injection needle 13a when the needle protection sleeve 3 is moved distally into the needle protection position.

As with the first embodiment, at least one wetted detection element 20.1, 20.2 will generate a signal for the electronic module 16 in order to inform the patient about the occurred error during the injection, i.e. during product discharge.

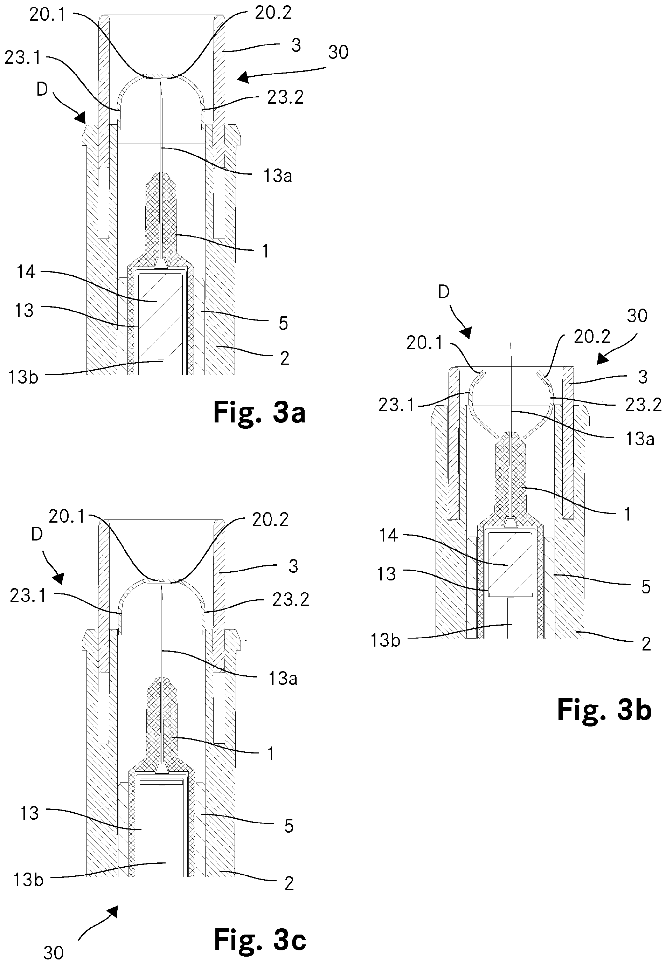

Figs. 3a, 3b and 3c schematically show longitudinal sectional views of a third embodiment of the autoinjector 30. Again, only the distal end D of the autoinjector 30 is shown in a simplified manner.

Contrary to the embodiments shown in the previous figures, the autoinjector according to the third embodiment comprises two support elements 23.1, 23.2 which at least partially close the distal end D of the autoinjector 30. In this first position of the two support elements 23.1, 23.2, a part of each support element 23.1, 23.2 is positioned distally in front of the injection needle 13a. In the embodiment shown, the two support elements 23.1, 23.2 are generally shaped as half arches which abut each other distally in front of the injection needle. In the area of contact of the two support elements 23.1, 23.2, a detection element 20.1, 20.2 is arranged on each of the support elements 23.1, 23.2 on the side thereof which is directed towards the housing 2, i.e. which is directed towards the proximal end of the autoinjector 30 in an initial or shipping state thereof. The two support elements 23.1, 23.2 are movably arranged on the housing 2 so that the two support elements 23.1, 23.2 may swivel and slide partially into the housing 2 (see Fig. 3b) in order to unsheathe a tip of the injection needle 13a. Otherwise, the reminder of the autoinjector 30 is substantially similar to the embodiments described above.

Fig. 3b shows the autoinjector 30 according to the fourth embodiment during dispensing of the liquid product 14, i.e. during product discharge. Prior to product discharge, the two ends of the support elements 23.1, 23.2 abutting each other are moved laterally relative to the injection needle 13a by a swivelling movement of the two support elements 23.1, 23.2 which also moves the support elements 23.1, 23.2 in the proximal direction slightly into the housing 2. At the end of this movement, the support elements 23.1, 23.2 are in a second position. This movement is coupled to the movement of the needle protection sleeve 3 in the proximal direction from the initial position into the actuated position. This coupling may be realized by an according mechanism (not shown) which provides a kinematic coupling of the movement of the needle protection sleeve 3 to the movement of the two support elements 23.1, 23.2. By the movement of the two support elements 23.1, 23.2 the tip of the injection needle 13a is unsheathed and freely protrudes distally from the needle protection sleeve 3. As with the previously described embodiments, the movement of the needle protection sleeve 3 causes the piston 13b to be moved into the dispensing direction in order to dispense the liquid product 14 out of the product container 13 and through the injection needle 13a for product discharge.

Fig. 3c shows the autoinjector 30 according to the third embodiment after product discharge, i.e. after the liquid product 14 has been dispensed from the product container 13. The needle protection sleeve 3 has been moved in the distal direction from the actuated position into the needle protection position. This movement of the needle protection shield 3 entails a movement of the support elements 23.1, 23.2 from the second position back into the first position, where a part of each of the support elements 23.1, 23.2 again is located distally in front of the injection needle 13a. At least a part of the liquid product 14 which has not been correctly injected to a patient during product discharge, either because of a wet injection or an air-shot, will contact or wet at least one of the detection elements 20.1, 20.2 either when the support elements 23.1, 23.2 are in the second position during product discharge or in the first position after product discharge.

## Claims

1. An autoinjector (30) for dispensing a liquid product, particularly a drug, comprising:
a) a housing (2) comprising a hollow space for housing a product container (13), preferably a syringe, the product container comprising a piston (13b), which is displaceable in a dispensing direction for dispensing a liquid product contained in the product container; the product container further being in fluid communication with a needle (13a) attached to a distal end thereof;
b) a propulsion means which acts on the piston during liquid product discharge;
c) a needle protection sleeve (3) axially moveable relative to the housing between a distal position and a proximal activated position, wherein the needle is radially surrounded by the needle protection sleeve in the distal position and the needle distally protrudes the needle protection sleeve in the second position;
**characterized in that**
d) the autoinjector comprises at least one detection element (20.1, 20.2) for detecting the presence of dispensed liquid product outside of the injection needle after liquid product dispense.

2. The autoinjector according to claim 1, **characterized in that** the at least one detection element is a liquid sensitive element which is in communication with an electronic module arranged in or on the housing.

3. The autoinjector according to claim 2, **characterized in that** the at least one detection element is a paper-based battery.

4. The autoinjector according to any of claims 1 to 3, **characterized in that** the at least one detection element is arranged on a movable arm element, said movable arm element being movable relative to the housing to move the at least one detection element towards the injection needle.

5. The autoinjector according to claim 4, wherein a movement of the needle protection sleeve causes movement of the arm element towards the injection needle.

6. The autoinjector according to any of claims 1 to 3, wherein the at least one detection element is arranged on the needle protection sleeve.

7. The autoinjector according to claim 6, **characterized in that** the at least one detection element is arranged on an inside wall of the needle protection sleeve at or near a distal end thereof.

8. The autoinjector according to any of claims 1 to 3, **characterized in that** the at least one detection element is arranged on at least one movable support element, the at least one movable support element being arranged within the needle protection sleeve at a distal end thereof and being movable from a first position, in which at least a part of the at least one movable support element is positioned distally in front of the injection needle, to a second position, in which the at least one movable support element is positioned laterally relative to the injection needle, and back to the first position.

9. The autoinjector according to claim 8, **characterized in that** the autoinjector comprises a mechanism which is configured to move the at least one movable support element from the first position to the second position when the needle protection sleeve is moved from its distal to its proximal position and to move the at least one movable support element from the second position back to the first position when the needle protection sleeve is moved back to its distal position.

10. The autoinjector according to any one of claims 2 to 9, **characterized in that** the electronic module comprises a wireless communication element which is configured to transmit a signal when the at least one detection element detects the presence of a liquid.
